# FASCICULE DE BREVET EUROPEEN

(11) **EP 3 019 028 B1**
(45) Date de publication et mention de la délivrance du brevet: **07.08.2019**
(21) Numéro de dépôt: 14738568.6
(22) Date de dépôt: 20.06.2014
(51) Int. Cl.: A23L 5/00, A23L 27/00, A61K 9/16, A61K 8/11

(54) **PROCÉDÉ DE FABRICATION DE GRANULÉS CONTENANT UN PRINCIPE ACTIF, À DLUO ET CHARGE EN PRINCIPE ACTIF OPTIMISÉES**
VERFAHREN ZUR HERSTELLUNG VON GRANULEN ENTHALTEND EINEN AKTIVEN VERBINDUNG, MIT VERBESSERTEN STABILITÄT UND GEHALT AN AKTIVEN
PROCESS FOR THE PRODUCTION OF GRANULES COMPRISING AN ACTIVE, HAVING IMPROVED SHELF LIFE AND LEVEL OF ACTIVE

(30) Priorité: 25.06.2013 FR 1356086
(43) Date de publication de la demande: 18.05.2016
(73) Titulaire: Expressions Aromatiques, 06370 Mouans Sartoux (FR)
(72) Inventeur: CARPENTIER, Vincent, F-06530 Saint Cezaire sur Siagne (FR); RAVEL, Bénédicte, F-06130 Grasse (FR)
(74) Mandataire: Lefevre-Groboillot, David André
(86) Numéro de dépôt international: PCT/FR2014/051551
(87) Numéro de publication internationale: WO 2014/207355

(56) Documents cités:
- EP-A1- 1 124 442
- WO-A2-2005/074699
- US-A- 4 820 534
- US-A- 5 603 971
- US-A1- 2005 220 981
- US-B1- 6 436 453

## Description

L'invention concerne un procédé de fabrication de granulés contenant au moins un principe actif par exemple aromatique, solubles et stables à température ambiante.

Des granulés de ce type peuvent être utilisés dans diverses applications : agroalimentaires comme les thés aromatisés, les boissons instantanées ; cosmétiques ou pharmaceutiques, dans chacune desquelles il est important qu'ils conservent leurs propriétés aussi longtemps que possible.

A cette fin, il est préférable que ces granulés présentent une température de transition vitreuse, température de passage d'un état vitreux, à un état amorphe caoutchouteux, bien plus élevée que la température ambiante pour que cette transition ne s'opère pas de façon incessante et que l'aspect, la texture et la structure des granulés ne soient pas modifiés.

Dans le procédé de fabrication de granulés de ce genre, connu notamment du document EP 1 124 442, ces granulés sont préparés par extrusion à partir d'un mélange d'une matrice glucidique avec un principe aromatique. Cette matrice est chauffée à une température d'extrusion puis passée en voie fondue à travers une filière d'extrusion pour enfin être découpée en sortie de filière. Pour doter les granulés d'une température de transition vitreuse supérieure à la température ambiante, une quantité d'eau, plus faible que celle jusqu'alors utilisée, est ajoutée au mélange de façon à limiter l'influence de l'eau sur la température de transition vitreuse des granulés, l'eau ayant tendance à diminuer cette température de transition vitreuse.

En outre, ce procédé permet une découpe des granulés directe en sortie d'extrudeuse, c'est-à-dire sans étape intermédiaire de séchage ou de refroidissement, car la matrice chauffée à la température d'extrusion est plastique et peut pour cette raison être mise en forme directement en sortie d'extrudeuse.

Ce résultat est atteint grâce à :
- l'utilisation d'une température d'extrusion telle que la matrice glucidique, de viscosité élevée, et le principe aromatique, de viscosité faible, ne se séparent pas durant l'extrusion bien que ces deux composés aient des viscosités différant l'une de l'autre de plusieurs ordres de magnitude,
- l'utilisation d'une pression d'extrusion telle que l'extrusion, qui se déroule nécessairement à une température supérieure à la température de transition vitreuse, puisse avoir lieu alors que la masse à l'état fondu est pourtant plastique et ce, sans addition d'un plastifiant non aqueux qui dans ce procédé, n'est pas désiré.

Le document WO2005074699 décrit un procédé de préparation de substances actives notamment arômes, parfums, huiles essentielles, extraits, médicaments, par extrusion dans une matrice fondue pouvant comprendre des 5 sucres et des maltodextrines.

L'invention propose un procédé de fabrication de granulés contenant au moins un principe actif, alternatif à celui décrit ci-dessus, et dont l'objectif principal est de pourvoir les granulés obtenus, d'une charge aromatique, cosmétique, pharmaceutique importante, d'une date limite d'utilisation optimale (DLUO) supérieure ou égale à 24 mois et dont le dosage en solution est optimisé.

A cet effet, l'invention concerne un procédé de fabrication de granulés contenant au moins un principe actif et stables à température ambiante, comprenant les étapes successives de :
(1) préparation d'une matrice dépourvue de principe actif par mélange sans adjonction d'eau, d'un véhicule d'encapsulation du principe actif et d'un plastifiant non aqueux compatible avec ce véhicule et avec le principe actif choisi, le véhicule d'encapsulation et le plastifiant étant présents dans des teneurs dotant la matrice d'une température de transition vitreuse supérieure à la température ambiante,
(2) transformation de la matrice dans une extrudeuse à une température d'extrusion pour obtenir une matrice en voie fondue amorphe,
(3) injection du principe actif au sein de la matrice en voie fondue, pour doter la masse en voie fondue d'un actif déterminé,
(4) extrusion de la masse en voie fondue dotée de l'actif au travers d'une filière et découpe à un point de sortie de filière de cette masse sous forme de granulés.

Selon l'invention, le plastifiant est le monopropylène glycol (MPG).

L'invention présente l'une ou l'autre des caractéristiques suivantes :
- ledit plastifiant est présent dans une teneur inférieure à une valeur prédéterminée à laquelle la température de transition vitreuse de la matrice devient supérieure à 40°C,
- ledit plastifiant est présent dans une teneur inférieure à 6% en poids sur le poids total des constituants des granulés,
- ledit plastifiant est présent dans une teneur supérieure à 1% en poids sur le poids total des constituants des granulés,
- le véhicule d'encapsulation comprend un monosaccharide et/ou un polysaccharide à chaîne courte seuls ou en mélange, associés à des maltodextrines,
- le principe actif est associé à un émulsifiant facilitant la dispersion du principe actif dans la masse fondue, et la dispersion du principe actif sous la forme d'une émulsion fine dans la solution aqueuse dans laquelle les granulés sont incorporés,
- l'émulsifiant présente un indice HLB compris entre 8 et 18
- l'émulsifiant comprend des polysorbates, des sucro-esters, des mono et di-glycérides d'acides gras, des émulsifiants riches en saponines et des lécithines seuls ou en mélange,
- le procédé comprend une étape de maintien en température de la masse fondue dotée d'un actif en sortie d'extrudeuse, entre 45 et 55°C.

L'invention concerne également des granulés dotés d'un principe actif, qui sont susceptibles d'être obtenus selon le procédé de l'invention et comprenant :
∘ de la maltodextrine et des monosaccharides ou polysaccharides à chaîne courte dans un rapport compris entre 80/20 et 20/80
∘ Entre 1 et 6% de MPG
∘ Entre 0,1 et 10% d'un principe actif

L'invention sera définie plus en détail dans la description qui va suivre, qui contient deux exemples de fabrication de granulés aromatisés, donnés à titre illustratif et non limitatif.

Le procédé selon l'invention a été mis au point dans le but de fabriquer des granulés aromatisés, présentant une charge aromatique élevée, une date limite d'utilisation optimale supérieure ou égale à 24 mois, se dissolvant sans trouble en milieux aqueux et présentant les caractéristiques suivantes :
Charge aromatique comprise entre 0,1 et 10 %, de
   préférence entre 2 à 10 %
Dosage optimal des granulés aromatisés dans la solution formée suite à l'ajout d'eau : 0.2 g.L⁻¹ à 1g.L⁻¹
Humidité résiduelle inférieure à 5%
Température de transition vitreuse supérieure à 40°C
DLUO supérieure ou égale à 24 mois
Arôme hydro-dispersible limpide dans la solution formée

Le principe de l'extrusion a été retenu car il permet une excellente encapsulation des principes aromatiques qui se retrouvent ainsi protégés de l'environnement extérieur et des causes de détérioration de saveur, d'odeur ou de goût.

Il consiste à insérer un mélange pulvérulent contenant un véhicule d'encapsulation, un plastifiant et un principe aromatique, de la composition désirée, au sein d'un appareil à extrusion, où ce mélange est chauffé à une température d'extrusion jusqu'à obtenir une masse fondue malléable, qui est introduite à l'entrée d'une filière dont le diamètre est proche de celui des granulés à obtenir, en étant toujours malléable, et découpée en granulés en sortie de filière, par un couteau sectionnant les fils formés en sortie de filière.

Ce procédé est mis en oeuvre au sein d'un appareil d'extrusion, tel qu'une extrudeuse bivis tel que celui commercialisé sous le nom de marque Clextral BC21.

Cette extrudeuse est équipée de façon connue, d'un doseur du véhicule d'encapsulation où le mélange pulvérulent est introduit, d'une bivis corotative qui véhiculera ce mélange vers la filière, au travers de différents modules équipés notamment de mécanismes de thermorégulation, de la filière elle-même positionnée en aval de la bivis, éventuellement d'un système de soufflage d'air thermo-régulé en sortie de filière et d'un appareil de découpe en aval de la sortie de la filière, ou en aval du système de soufflage si l'extrudeuse en est pourvu.

Pour fabriquer des granulés remplissant le cahier des charges ci-dessus, l'invention concerne un procédé mis en oeuvre au sein d'une extrudeuse bivis corotative et comprend les étapes successives de :
(1) préparation d'une matrice dépourvue de principe actif par mélange sans adjonction d'eau, d'un véhicule d'encapsulation du principe actif et d'un plastifiant non aqueux compatible avec ce véhicule et avec le principe actif choisi, le véhicule d'encapsulation et le plastifiant étant présents dans des teneurs dotant la matrice d'une température de transition vitreuse supérieure à la température ambiante,
(2) transformation de la matrice dans une extrudeuse à une température d'extrusion pour obtenir une matrice en voie fondue amorphe,
(3) injection du principe actif associé à un émulsifiant compatible avec ce principe actif et avec le plastifiant, au sein de la matrice en voie fondue, pour doter la masse en voie fondue d'un actif déterminé,
(4) extrusion de la masse en voie fondue dotée de l'actif au travers d'une filière et découpe à un point de sortie de filière de cette masse sous forme de granulés.

Le plastifiant choisi est le monopropylène glycol (MPG).

L'extrudeuse convenant à la mise en oeuvre de ce procédé devra être pourvue d'un point d'entrée pour le principe actif en fin de vis et en amont de la filière.

Ainsi, une extrudeuse de ce type comprendra successivement : une zone d'incorporation des poudres constituant le véhicule d'encapsulation, une zone d'incorporation du plastifiant ; le long de la bivis une zone de transformation de la matrice formée par le véhicule d'encapsulation mélangé au plastifiant en une matrice à l'état visqueux, une zone d'incorporation de l'arome dans la matrice à l'état visqueux, une ultime zone de mélange de l'arome dans la matrice à l'état visqueux ; la filière d'extrusion et l'outil de découpe des granulés en sortie de cette filière.

Les étapes successives du procédé selon l'invention ont été mises au point pour éviter de soumettre le principe actif à des traitements thermiques susceptibles d'en détériorer les propriétés.

Le procédé selon l'invention prévoit ainsi de ne l'injecter qu'en toute fin du processus (étape 3) c'est-à-dire lorsque le mélange pulvérulent a été suffisamment transformé pour se présenter sous la forme d'une masse fondue, et de ne procéder à aucune addition d'eau dans le mélange initial, afin d'éviter toute opération ultérieure de séchage et l'apparition de poches de vapeur. Cette injection s'effectuera à un point le long de la bivis qui soit le plus proche de la filière d'extrusion pour garantir une dispersion homogène du principe aromatique au sein de la matrice à l'état fondu à l'issue de la dernière zone de mélange, minimisant ainsi le temps d'exposition de ce principe actif à la température d'extrusion. Le temps de séjour du principe aromatique thermosensible au sein de l'extrudeuse est ainsi optimisé. A titre d'exemple, l'injection du principe actif sera réalisée juste avant la dernière zone de mélange de la bivis, puis cette masse fondue sera introduite au sein de la filière.

### Véhicule d'encapsulation

Le véhicule d'encapsulation qui est associé au plastifiant afin de constituer la matrice non aromatisée, comprend des carbohydrates : un mélange de polysaccharides à chaînes courtes tels que le saccharose, et de maltodextrines par exemple d'indice DE 9 ou 12 couramment utilisés dans le domaine des arômes extrudés. Une précaution particulière sera toutefois prise au niveau de la sélection de la maltodextrine et de son indice pour garantir que les granulés obtenus aient une teneur en glucose inférieure à 0.5%, valeur en dessous de laquelle il a pu être observé une absence totale de problème de collage de la matrice sur la bivis de l'extrudeuse, ou sur le couteau en sortie.

Si les polysaccharides à chaîne courte ont été particulièrement préférés, tels que le mono ou le disaccharide, c'est qu'ils permettent une meilleure rétention du principe aromatique.

Quant aux maltodextrines, leur sélection a été opérée car elles augmentent la température de transition vitreuse au-delà de 40°C ou proche de cette valeur, pour garantir la stabilité des granulés à température ambiante.

Le rapport Maltodextrine/mono ou polysaccharides dans la matrice est compris entre 80/20 et 20/80, mais toujours limité par la contrainte de pourvoir les granulés obtenus d'une teneur en glucose absolument inférieure à 0.5% par extrême précaution afin d'éviter tout problème de collage.

Le rapport préféré étant de l'ordre de 55/45.

La matrice doit être, bien entendu, compatible avec le plastifiant choisi. Une granulométrie inférieure à 1 mm sera préférée : les agrégats de carbohydrates doivent être suffisamment petits pour entrer en fusion rapidement en présence de MPG ou d'un autre solvant du même type. Dans le cas contraire, quelques morceaux pourraient boucher les trous de la filière et empêcher une production continue (ces derniers ayant un diamètre compris entre 0,5 et 2 mm).

### Plastifiant

Le plastifiant permet la transformation de ce véhicule notamment par chauffage, en une masse malléable brillante sans agrégat.

Pour remplacer l'eau généralement utilisée comme plastifiant de ce type de véhicules d'encapsulation et qui implique des étapes contraignantes et susceptibles d'entraîner l'évaporation du principe aromatique (phases de séchage et de dégazage de vapeur d'eau) ou de provoquer un vieillissement prématuré du principe aromatique, le procédé selon l'invention prévoit l'utilisation d'un plastifiant liquide non aqueux jouant le rôle de solvant en ayant pour fonction de rendre compatibles le principe aromatique plutôt hydrophobe, avec le véhicule d'encapsulation plutôt hydrophile, et favoriser ainsi la dispersion du principe aromatique dans la masse fondue juste avant le passage au sein de la filière.

Sa présence évite le phénomène de ségrégation entre le véhicule d'encapsulation et le principe actif, qui intervient généralement lors du chauffage puisque ces deux composés présentent des viscosités différant l'une de l'autre de plusieurs ordres de grandeur, puisqu'il abaisse la viscosité du véhicule d'encapsulation.

L'addition d'eau ayant été proscrite, et le plastifiant choisi ayant une température d'ébullition élevée, la formation de poches de vapeur d'eau et d'arôme est évitée, et la fabrication continue, sans interruption de dégazage, est possible.

Pour en outre garantir un aspect identique aux granulés obtenus quelles que soient les fluctuations de température ambiante, la nature et la concentration du plastifiant sont choisies pour conférer à ces granulés une température de transition vitreuse nettement supérieure à celle de la température ambiante de 20-25°C, de préférence supérieure à 40°C.

Le monopropylène glycol (MPG) a été choisi comme plastifiant non seulement pour sa haute température d'ébullition mais aussi pour son importante faculté à plastifier le véhicule d'encapsulation, ce qui permet de diminuer la température d'extrusion appliquée au cours du procédé et diminue ainsi les contraintes thermiques subies par le principe actif lors de son introduction dans la masse fondue portée à cette température, et pour sa faculté de dispersion d'un principe aromatique dans le type de véhicule d'encapsulation choisi. Le MPG se présente sous forme de liquide non aqueux et joue notamment le rôle de solvant comme expliqué précédemment.

On a pu déterminer que le monopropylène glycol (MPG) était le solubilisant le plus efficace sur le plus grand nombre de types de principes aromatiques différents, notamment meilleur que le glycérol. En effet, les principaux esters, alcools, acides, aldéhydes, cétones et composés hétérocycliques, qui composent la majeure partie des principes aromatiques, sont solubles dans le MPG.

Mais sa tendance à abaisser la température de transition vitreuse en dessous de 40°C voire de 20°C lorsqu'il est incorporé dans des proportions trop importantes à un véhicule d'encapsulation à base de carbohydrate, n'imposait pas la sélection du MPG à l'évidence, le risque étant de devoir l'utiliser dans des proportions plus faibles pour garantir qu'une Tg supérieure à 40°C soit atteinte, avec l'inconvénient de ne pas atteindre une plastification totale de la matrice, qui serait alors pourvue des agrégats de carbohydrates susceptibles de boucher la filière, et d'occasionner des arrêts de production, ce qui n'est pas admissible. Et la solution d'augmenter alors la température d'extrusion pour plastifier ces agrégats, n'est pas non plus souhaitée puisqu'elle est susceptible d'endommager la matrice, qui se retrouve grisée, entachée de points de carbohydrates brûlés.

Pourtant, de façon surprenante, une teneur optimale de MPG, garantissant à la fois une Tg inférieure à 40°C, et une plastification totale de la matrice a pu être déterminée.

Cette teneur en MPG est comprise entre 1 et 6% en poids total des ingrédients constitutifs des granulés.

En effet, il a pu être déterminé expérimentalement que le MPG utilisé dans une teneur de 5% en poids d'une matrice contenant des maltodextrines et du saccharose dans une teneur 55/45, contribuait à former en sortie d'extrudeuse, des granulés ayant une température de transition vitreuse proche de 50°C, valeur tout à fait correcte.

Et lorsqu'il est utilisé à une concentration de 10% en poids au sein d'une matrice du même type, les granulés obtenus ne présentent qu'une Tg de 30°C, c'est-à-dire une valeur trop faible pour que ces granulés soient suffisamment stables à température ambiante.

L'utilisation du MPG dans la teneur préconisée en dessous d'une valeur de seuil (6% en poids des ingrédients constitutifs des granulés), a donc permis d'obtenir une plastification complète de la matrice et de doter les granulés d'une température de transition vitreuse supérieure à 40°C.

L'utilisation du MPG a en outre évité la génération de poche de vapeur, a permis d'abaisser la température d'extrusion à 86°C voire à une valeur inférieure, et de remplir le cahier des charges précité.

En effet, le haut point d'ébullition du MPG (188°C) très largement au dessus de la température d'extrusion nécessaire et suffisante de 86°C, empêche toute formation de poches de gaz qui se forment usuellement avec un plastifiant à faible température d'ébullition tel que l'eau.

La température d'extrusion abaissée à 86°C voire en dessous, a encore accru la préservation des propriétés du principe aromatique injecté dans la masse fondue à cette température.

Ces degrés Celsius gagnés par rapport à des procédés d'extrusion existants tels que celui cité dans la partie de description consacrée à l'état de la technique, ont toute leur importance compte tenu des exigences fixées dans le cadre de l'invention pour la charge aromatique des granulés.

Cet effet a été observé pour une teneur en plastifiant égale ou supérieure à 1% en poids des ingrédients entrant dans la composition des granulés.

On fixe pour ces motifs, la teneur optimale en plastifiant comprise entre 1 et 6% en poids des ingrédients entrant dans la composition des granulés.

La température d'extrusion relativement faible conduit à l'obtention en sortie de filière d'extrusion, d'un boudin à également relativement faible température et donc susceptible de se solidifier rapidement. Pour que cette solidification se fasse dans des conditions optimales, un module de maintien de ces fils en température (par exemple entre 45 et 55°C) sera prévu entre la sortie de la filière et le couteau, température, permettant la formation d'une enveloppe externe solide pour les fils, tandis que le coeur des fils reste fondu, favorisant une découpe nette des granulés.

Le plastifiant choisi, par son importante compatibilité avec le principe aromatique, facilite la dispersion de ce dernier dans la masse fondue avant l'extrusion au travers de la filière et permet enfin d'optimiser la charge aromatique et son dosage dans la solution aqueuse.

### Principe aromatique

L'ajout du principe aromatique augmentant la Tg des granulés obtenus, c'est bien la teneur en plastifiant qu'il faudra optimiser pour garantir qu'une Tg supérieure à 40°C soit atteinte.

Cette dispersion du principe aromatique dans la masse fondue est en outre facilitée par l'émulsifiant sur lequel est monté le principe aromatique, bien que cet émulsifiant ait comme objectif premier de faciliter la dispersion du principe aromatique sans trouble dans la solution aqueuse dans laquelle les granulés seront incorporés.

Le plastifiant utilisé dans la teneur qui a été déterminée en fonction de la Tg devant être atteinte par les granulés, est en effet susceptible de ne pas permettre à lui seul une dispersion sans trouble du principe aromatique en milieux aqueux.

L'émulsifiant incorporé à cette occasion doit être compatible avec le principe aromatique et avec le plastifiant.

Idéalement, cet émulsifiant peut présenter un indice HLB compris entre 8 et 18, de préférence supérieur à 10.

Les polysorbates, les lécithines, ou les extraits riches en saponines qui sont des émulsifiants ne nécessitant pas d'hydratation préalable et sont parfaitement miscibles avec la plupart des principes aromatiques, conviennent particulièrement pour atteindre les objectifs de l'invention.

Ces émulsifiants sont intégrés en mélange avec le principe aromatique sous pression, juste avant la dernière zone de mélange de la bivis, pour optimiser la dispersion du principe aromatique dans la matrice puisqu'ils évitent les phénomènes de déphasage et de vaporisation, l'émulsifiant permettant une meilleure dispersion des composés hydrophobes comme les terpènes dans la matrice hydrophile composée de carbohydrates et de solvant polaire.

D'autres émulsifiants peuvent être utilisés comme des sucro-esters, des mono et di-glycérides d'acides gras seuls ou en mélange.

L'émulsifiant sera utilisé dans une teneur respectant la législation en vigueur, par exemple inférieure à 5% en poids des ingrédients constitutifs des granulés, et supérieure à 0.1% en poids pour remplir leur fonction de solubilisant du principe aromatique. Il permet la formation d'une émulsion fine du principe actif dans la solution aqueuse dans laquelle les granulés sont introduits et évitent de ce fait la formation d'un trouble.

Il est à noter que lorsque les granulés sont destinés à être incorporés dans des préparations pour aliments solides, tels que des biscuits, dans lesquels par définition la notion de trouble de solution ne se présentera pas, il ne sera pas indispensable que le principe aromatique soit associé à l'émulsifiant avant son injection dans la masse en voie fondue. Dans cette application plus particulière, l'utilisation du glucose comme source de monosaccharide sera également évitée, ce dernier rendant la matrice plus collante et engendrant donc des difficultés au cours de la granulation en sortie d'extrudeuse. Les principes aromatiques contenus dans ces granulés destinés à l'industrie de la biscuiterie, seront répartis de manière homogène dans la pâte à biscuit grâce à l'action mécanique du pétrissage.

Les principes aromatiques pouvant être encapsulés selon le procédé de l'invention, sont ceux des catégories définies dans le règlement CE 1334/2008 du Parlement Européen et du conseil du 16 décembre 2008, ou un mélange de plusieurs de ces catégories :
- substances aromatisantes
- substances aromatisantes naturelles
- préparations aromatisantes
- arômes obtenus par traitement thermique
- arômes de fumées
- précurseurs d'arômes
- autres arômes
- ingrédients alimentaires possédant des propriétés aromatisantes

Bien entendu, d'autres principes actifs respectant d' autres législations pourront faire l'objet d'encapsulation par le procédé selon l'invention.

Il est prévu d'utiliser le principe aromatique dans une teneur comprise entre 0.1 et 10% en poids des ingrédients constitutifs des granulés. La marge haute pouvant toutefois être dépassée puisqu'une teneur élevée en principe aromatique ne diminue pas la Tg des granulés obtenus. Il faudra toutefois adapter la teneur en émulsifiant et en plastifiant pour disperser cet arome dans la voie fondue.

De même, ce procédé pourra être utilisé pour l'encapsulation de principes actifs pharmaceutiques, de parfums, de produits cosmétiques et d'hygiène, applications pour lesquelles la courte exposition du principe actif à la température d'extrusion et la faible valeur de cette température d'extrusion sont d'autant plus appréciables que les principes actifs sont thermosensibles.

On pourra aussi appliquer l'invention à la fabrication de granulés comprenant des compléments alimentaires.

Exemples de réalisation de granulés aromatiques selon l'invention:
1) Granulés d'arôme pêche (% en poids):
   Matrice poudre :
      Saccharose : 41.2
      Maltodextrine DE9 : 50.3
   Plastifiant :
      MPG E1520 : 5
   Base aromatique pêche : 2.92
   Emulsifiant :
      Polysorbate E432 : 0.58
      T d'extrusion : 86°C (max)
      P d'extrusion : < 15 bars en butée
      Tg des granulés : 47°C
2) Granulés d'arôme orange (% en poids):
   Matrice poudre :
      Saccharose : 42.2
      Maltodextrine DE9 : 51.6
   Plastifiant :
      MPG E1520 : 2.1
   Base aromatique orange : 2.1
   Emulsifiant :
      Polysorbate E432 : 2
      T d'extrusion : 85°C (max)
      P d'extrusion : <15bars en butée
      Tg des granulés : 61°C

Dans les deux exemples, la taille des particules huileuses après dispersion en milieu aqueux est approximativement comprise entre 10 et 500 nm.

## Revendications

1. Procédé de fabrication de granulés contenant au moins un principe actif et stables à température ambiante, comprenant les étapes successives de :
(1) préparation d'une matrice dépourvue de principe actif par mélange sans adjonction d'eau, d'un véhicule d'encapsulation du principe actif et d'un plastifiant non aqueux compatible avec ce véhicule et avec le principe actif choisi, le véhicule d'encapsulation et le plastifiant étant présents dans des teneurs dotant la matrice d'une température de transition vitreuse supérieure à la température ambiante,
(2) transformation de la matrice dans une extrudeuse à une température d'extrusion pour obtenir une matrice en voie fondue amorphe,
(3) injection du principe actif au sein de la matrice en voie fondue, pour doter la masse en voie fondue d'un actif déterminé,
(4) extrusion de la masse en voie fondue dotée de l'actif au travers d'une filière et découpe à un point de sortie de la filière de cette masse sous forme de granulés,
dans lequel procédé le plastifiant est le monopropylène glycol.

2. Procédé selon la revendication précédente, dans lequel ledit plastifiant est présent dans une teneur inférieure à une valeur prédéterminée à laquelle la température de transition vitreuse des granulés devient supérieure à 40°C.

3. Procédé selon l'une des revendications précédentes,
dans lequel ledit plastifiant est présent dans une teneur inférieure à 6% en poids sur le poids total des constituants des granulés.

4. Procédé selon l'une des revendications précédentes,
dans lequel ledit plastifiant est présent dans une teneur supérieure à 1% en poids sur le poids total des constituants des granulés.

5. Procédé selon l'une des revendications précédentes, dans lequel le véhicule d'encapsulation comprend un monosaccharide ou des polysaccharides seuls ou en mélange avec, et des maltodextrines.

6. Procédé selon l'une des revendications précédentes, dans lequel le principe actif est monté sur un émulsifiant facilitant la dispersion du principe actif dans la masse fondue, et la dispersion du principe actif sous la forme d'une émulsion fine dans la solution aqueuse dans laquelle les granulés sont incorporés.

7. Procédé selon la revendication 6, dans lequel l'émulsifiant présente un indice HLB compris entre 8 et 18.

8. Procédé selon la revendication 7, dans lequel l'émulsifiant comprend des polysorbates, des sucro-esters, des mono et di-glycérides d'acides gras, des émulsifiants riches en saponines et des lécithines seuls ou en mélange.

9. Procédé selon l'une des revendications précédentes comprenant une étape de maintien en température de la masse fondue dotée d'un actif en sortie d'extrudeuse, entre 45 et 55°C.

10. Granulés dotés d'un actif susceptibles d'être obtenus selon le procédé de l'une quelconque des revendications 1 à 9, comprenant :
- de la maltodextrine et des monosaccharides ou des polysaccharides à chaînes courtes tels que le disaccharide dans un rapport compris entre 80/20 et 20/80
- entre 1 et 6% de monopropylène glycol
- entre 0,1 et 10% d'un principe actif.

## Patentansprüche

1. Verfahren zur Herstellung von Granulaten, enthaltend mindestens einen Wirkstoff und stabil bei Umgebungstemperatur, umfassend die folgenden Schritte:
(1) Herstellen einer Matrix, die keinen Wirkstoff aufweist, durch Mischen, ohne Zugabe von Wasser, eines Einkapselungsträgers des Wirkstoffs und eines nicht wässrigen Weichmachers, der mit diesem Träger kompatibel ist, und mit dem ausgewählten Wirkstoff, wobei der Einkapselungsträger und der Weichmacher, die in den Gehalten anwesend sind, die Matrix mit einer Glaswandlungstemperatur dotieren, die höher als die Umgebungsgtemperatur ist,
(2) Umwandeln der Matrix in einem Extruder bei einer Extrusionstemperatur, um eine Matrix durch amorphe Schmelzformung zu erhalten,
(3) Injizieren des Wirkstoffs in das Innere der Matrix durch Schmelzformung, um die Masse durch Schmelzen mit einem bestimmten Wirkstoff zu dotieren,
(4) Extrudieren der Masse durch Schmelzformung, die mit dem Wirkstoff dotiert ist, mit Hilfe einer Düse und Schneiden an einem Ausgangspunkt der Düse dieser Masse in Form von Granulaten, wobei in diesem Verfahren der Weichmacher Monopropylenglykol ist.

2. Verfahren nach dem vorhergehenden Anspruch, wobei der Weichmacher mit einem Gehalt von weniger als einem vorbestimmten Wert vorhanden ist, bei dem die Glaswandlungstemperatur der Granulate höher als 40 °C wird.

3. Verfahren nach einem der vorhergehenden Ansprüche, wobei der Weichmacher mit einem Gehalt von weniger als 6 Gew% zum Gesamtgewicht der Bestandteile der Granulate vorhanden ist.

4. Verfahren nach einem der vorhergehenden Ansprüche, wobei der Weichmacher mit einem Gehalt von mehr als 1 Gew% zum Gesamtgewicht der Bestandteile der Granulate vorhanden ist.

5. Verfahren nach einem der vorhergehenden Ansprüche, wobei, der Einkapselungsträger ein Monosaccharid oder Polysaccharide alleine oder in Mischungen und Maltodextrine umfasst.

6. Verfahren nach einem der vorhergehenden Ansprüche, wobei der Wirkstoff auf einen Emulgator montiert ist, wodurch die Dispersion des Wirkstoffs in der geschmolzenen Masse und die Dispersion des Wirkstoffs in Form einer feinen Emulsion in der wässrigen Lösung, in der die Granulate eingeschlossen sind, erleichtert wird

7. Verfahren nach Anspruch 6, wobei der Emulgator einen HLB-Index zwischen 8 und 18 aufweist.

8. Verfahren nach Anspruch 7, wobei der Emulgator Polysorbate, Zuckerester, Mono- und Diglyceride von Fettsäuren, Emulgatoren reich an Saponinen und Lecithine alleine oder in Mischungen umfasst.

9. Verfahren nach einem der vorhergehenden Ansprüche, umfassend einen Schritt des Beibehaltens der Temperatur der geschmolzenen Masse, dotiert mit einem Wirkstoff, am Ausgang des Extruders, zwischen 45 und 55 °C.

10. Granulate, dotiert mit einem Wirkstoff, der nach dem Verfahren eines der Ansprüche 1 bis 9 erhalten werden kann, umfassend:
- Maltodextrin und Monosaccharide oder kurzkettige Polysaccharide wie z. B. Disaccharid in einem Verhältnis im Bereich zwischen 80/20 und 20/80
- zwischen 1 und 6 % Monopropylenglykol
- zwischen 0,1 und 10 % Wirkstoff.

## Claims

1. A process for the production of granules containing at least one active ingredient and that are stable at ambient temperature, comprising the following successive steps:
(1) preparing a matrix with no active ingredient by mixing without any addition of water, of a vehicle for encapsulating the active ingredient and a nonaqueous plasticizer compatible with this vehicle and with the selected active ingredient, the encapsulating vehicle and the plasticizer being present in content levels equipping the matrix with a glass transition temperature higher than the ambient temperature,
(2) transforming the matrix in an extruder at an extrusion temperature to obtain a matrix in an amorphous molten path,
(3) injecting active ingredient within the matrix in the molten path, to equip the mass in the molten path with a determined active ingredient,
(4) extruding the mass in the molten path equipped with the active ingredient through a nozzle and cutting this mass at an exit path from the nozzle in the form of granules,
in which process the plasticizer is monopropylene glycol.

2. The process according to the preceding claim, wherein said plasticizer is present in a content level below a predetermined value at which the glass transition temperature of the granules becomes higher than 40°C.

3. The process according to one of the preceding claims, wherein said plasticizer is present in a content level below 6% by weight of the total weight of the components of the granules.

4. The process according to one of the preceding claims, wherein said plasticizer is present in a content level above 1% by weight of the total weight of the components of the granules.

5. The process according to one of the preceding claims, wherein the encapsulating vehicle comprises a monosaccharide or polysaccharides alone or in mixture with, and maltodextrins.

6. The process according to one of the preceding claims, wherein the active ingredient is mounted on an emulsifier facilitating the dispersion of the active ingredient in the molten mass, and the dispersion of the active ingredient in the form of a fine emulsion in the aqueous solution in which the granules are incorporated.

7. The process according to claim 6, wherein the emulsifier has an HLB index comprised between 8 and 18.

8. The process according to claim 7, wherein the emulsifier comprises polysorbates, sucroesters, mono- and di-glycerides of fatty acids, emulsifiers rich in saponins and lecithins alone or in mixture.

9. The process according to one of the preceding claims comprising a step for maintaining the temperature of the molten mass provided with an active ingredient leaving the extruder, between 45 and 55°C.

10. Granules provided with an active ingredient capable of being obtained according to the process of any one of claims 1 to 9, comprising:
- maltodextrin and short-chain monosaccharides or polysaccharides such as the disaccharide in a ratio of between 80/20 and 20/80
- between 1 and 6% monopropylene glycol
- between 0.1 and 10% of an active ingredient.
